# EUROPEAN PATENT SPECIFICATION

(11) **EP 1 893 758 B1**
(45) Date of publication and mention of the grant of the patent: **24.12.2014**
(21) Application number: 06747938.6
(22) Date of filing: 20.06.2006
(51) Int. Cl.: C12N 15/11, C07H 21/04

(54) **HYBRIDIZATION-STABILIZING CONSTRUCT**
HYBRIDISIERUNG STABILISIERENDES KONSTRUKT
CONSTRUCTION GÉNIQUE STABILISANT UNE HYBRIDATION

(30) Priority: 20.06.2005 US 692844 P
(43) Date of publication of application: 05.03.2008
(73) Proprietor: Oligomer Sciences AB, 182 60 Djursholm (SE)
(72) Inventor: SMITH, Edvard, S-112 42 Stockholm (SE); SVAHN, Mathias, S-120 66 Stockholm (SE); GE, Rongbin, Quincy, MA 02169 (US)
(74) Representative: Holmberg, Martin Tor
(86) International application number: PCT/SE2006/000746
(87) International publication number: WO 2006/137786

(56) References cited:
- WO-A1-99/55916
- WO-A1-03/091455
- US-A- 5 527 899
- US-B1- 6 372 427
- GE RONGBIN ET AL: "Zorro locked nucleic acid induces sequence-specific gene silencing." THE FASEB JOURNAL : OFFICIAL PUBLICATION OF THE FEDERATION OF AMERICAN SOCIETIES FOR EXPERIMENTAL BIOLOGY JUN 2007 LNKD- PUBMED:17314142, vol. 21, no. 8, June 2007 (2007-06), pages 1902-1914, XP002599884 ISSN: 1530-6860
- GE RONGBIN ET AL: "Sequence-specific inhibition of RNA polymerase III-dependent transcription using Zorro locked nucleic acid (LNA)." THE JOURNAL OF GENE MEDICINE JAN 2008 LNKD- PUBMED:18023071, vol. 10, no. 1, January 2008 (2008-01), pages 101-109, XP002599885 ISSN: 1099-498X
- LUNDIN K.E. ET AL.: 'Cooperative strand invasion of supercoiled plasmid DNA by mixed linear PNA and PNA-peptide chimeras' BIOMOLECULAR ENGINEERING vol. 21, 2004, pages 51 - 59, XP004505218
- LING J Q ET AL: "Long-range DNA interactions are specifically altered by locked nucleic acid-targeting of a CTCF binding site", BBA - GENE REGULATORY MECHANISMS, ELSEVIER, AMSTERDAM, NL, vol. 1809, no. 1, 1 January 2011 (2011-01-01), pages 24-33, XP027590837, ISSN: 1874-9399 [retrieved on 2011-01-01]

## Description

The present invention concerns the field of genetic engineering, and in particular a construct for stabilizing hybridization to double stranded DNA or RNA through trans-stabilizing-hybridization between both strands.

### Background

Abnormal gene expression is associated with many human disorders, such as malignant tumors. Silencing of specific gene expression could have a tremendous impact on the treatment of these diseases. Small molecules that bind DNA duplex in a sequence-specific manner are attractive candidates for genetic manipulation and have the potential to be developed into gene-based therapeutics.

Locked Nucleic Acid (LNA) bases contain a bridging methylene carbon between the 2' and 4' positions of ribose ring(1, 2). This constraint preorganizes the oligonucleotide backbone and can increase Tₘ values by as much as 10°C per LNA base replacement. LNA resembles natural nucleic acids with respect to Watson-Crick base pairing. LNA bases are introduced by standard DNA/RNA synthesis protocols, so LNA can be synthesized as pure LNA oligomers or mixed LNA/DNA oligomers. Moreover, LNAs have been demonstrated to be very efficient in binding to complementary nucleic acids and to be active antisense agents in vitro and in cultured mammalian cells(3-5), and also as decoys(6), aptamers(7), LNAzymes(8), and DNA correcting agents(9). However, LNA has never been shown to be very effective as small molecules for strand invasion in the DNA duplex nor to be very effective as anti-gene agents either *in vitro* or *in vivo.*

Peptide Nucleic Acid (PNA) is an oligonucleotide analog with a neutral pseudopeptide backbone consisting of repeating *N*- (2-amino-ethyl)-glycine units(10). PNA is capable of sequence-specific recognition of DNA and RNA obeying the Watson-Crick base pairing rulers(11), and the hybrid complexes exhibit extraordinary thermal stability and unique ionic strength effects. It may also recognize duplex homopurine sequences of DNA to which it binds by strand invasion, forming an extremely stable PNA-DNA-PNA triplex with a looped-out DNA strand(12-14). Strand invasion is most efficient when pyrimidine PNAs are connected by flexible linker to form a bisPNA in which one PNA strand hybridizes to DNA by Watson-Crick base paring, while another binds by Hoogsteen base paring. PNA appears to be resistant to both cellular nucleases and proteases(15). Moreover, recently it has been reported that tail-clamp PNA widened the application for strand invasion and can bind to duplex DNA and inhibit transcription *in vitro* (16, 17). These properties of PNA should have been advantageous for use as anti-gene molecules. Unfortunately, to date, although it was well established from in-vitro cell-free experiments that triplex invasion complexes are effective inhibitors of transcription initiation as well as transcription elongation, especially when positioned on the template strand (16-19), the present inventors are not aware of any direct proof that these triplex invasion complexes can succeed in arresting transcription elongation in cultured mammalian cells.

WO 96/02558 A1 (PCT/US95/09084) concerns a construct, based on the inclusion of DNA analogues with a non-cyclic backbone, i.e. Peptide Nucleic Acid. Further, at least one of the oligonucleotides exhibits Hoogsteen binding. It is also clearly stated that the linkage between the two oligonucleotides and/or peptides is covalent. One major advantage of the construct according to the present invention is the base pairing linkage.

WO 03/091455 A1 (PCT/LTS03/12480) presents a version of a Z-shaped LNA, but with the important difference of having one strand binding Watson-Crick and the second Hoogsteen. The two strands can bind the same (cis) or different (trans) strands. The Hoogsteen binding strand can bind either at the same position or at another position, creating Z-like structure but with Hoogsteen.

WO 95/01369 A1 (PCT/IB94/00211) mainly discloses the use of PNA as oligonucleotides for decoy, antisense, fluorescent probes and binding/blocking DNA binding proteins. Base pairing through Watson-Crick is not described as a possible linker between the two oligonucleotides. The linker is covalent or non-covalent and is added to keep the two oligonucleotides in place for hydrogen base pairing, generating double stranded structure. Furthermore, only linear backbones are addressed.

WO 96/32474 discloses a construct for inhibiting gene expression wherein at least two oligonucleotides of inverted polarity are covalently linked to one another,said oligonucleotides binding to both strands of the target duplex.

WO 99/22018 A2 (PCT/US98/22785) describes the use of analogue probes, but restricted to detection, identification and quantification. There does not seem to be any mentioning of means of functionality (gene silencing, gene transfer etc.). The document does describe partial hybridization between two oligonucleotides, possible to be mistaken for a Z-shaped intra-linkage according to the present invention. The disclosure is rather focused on achieving quenching between the two oligonucleotides when hybridized, giving a fluorescent signal when separated (the sample lights up when the correct sequence is present and the oligonucleotide-oligonucleotide hybridization is competed out).

The present inventors have found that bisPNA could not cause gene down-regulation with a reasonable molar excess, although several reports have shown that it could work *in vitro.* In this study, the present inventors tried to investigate the possibility to block transcription of a gene via harboring of tail-clamp PNA to template strand in cultured mammalian cells. Only when an enormous amount of PNA (150-fold molar excess) was added to plasmid with six PNA binding sites, decrease in protein expression was seen. This phenomenon could be due to formation of big complexes around multiple binding sites by binding of an enormous amount of PNA.

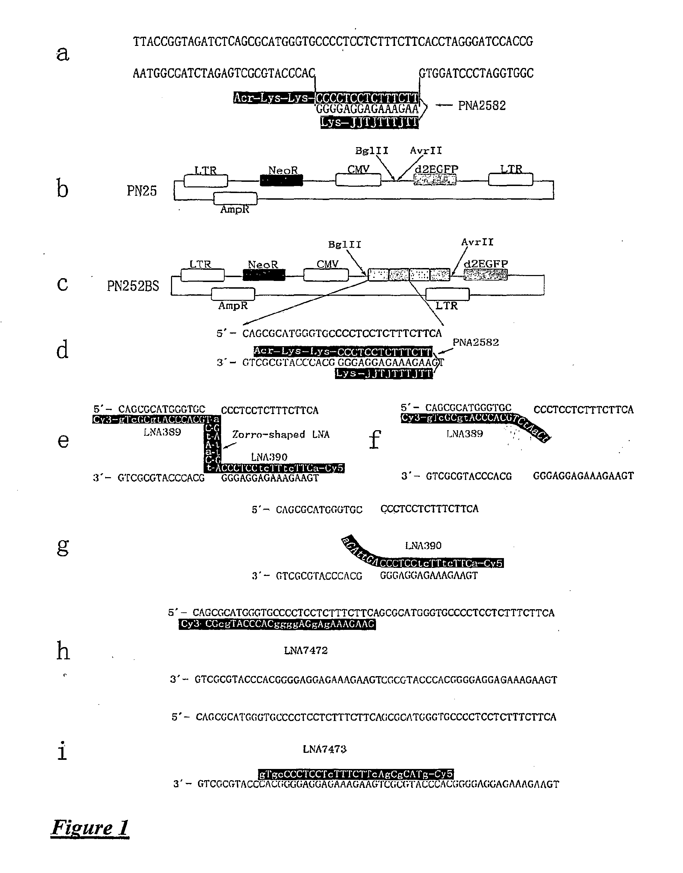

However, the present inventors generated a novel antigene reagent "Z-shaped LNA", in which one LNA strand binds to coding strand, another strand binds to opposite template strand, the two linear LNA strands were connected by a bridge containing seven bases pairing (Fig.1). The present inventors have verified that the Z-shaped LNA induced effective strand invasion to DNA duplex and in cultured mammalian cells, the "Z-shaped LNA" induced very powerful gene down-regulation (95%-100%) as an antigene reagent, whereas both linear LNA and bis-PNA failed.

### Summary of the invention

The present inventors make available a construct having the capacity to hybridize to double stranded DNA in a trans fashion to affect transcription is produced from at least two oligonucleotides, connected to each other, wherein the oligonucleotides are connected to one another through sequence specific Watson-Crick base pairing.

The invention and embodiments thereof is further defined in the attached claims, incorporated herein by reference.

### Description of the drawings

The present invention will be described in closer detail in the following description and non-limiting examples, with reference to the attached drawings in which

**Fig. 1** shows a schematic representation of the target site and sequence hybridized by LNA and PNA oligomers. The oligonucleotide containing LNA and PNA target sites was cloned into the position between *Bgl*II and *Avr*II of the plasmid PN25. **a,** The 59-mer duplex oligonucleotides hybridized to tail-clamp bisPNA, PNA2582. **b,** PN25 plasmid construct **c.** PN252BS plasmid construct **d.** PNA2582 and its corresponding target site **e.** Z-shaped LNA construct, in which a 14-mer arm (LNA389) is bound to coding strand, while another 16-mer arm (LNA390) is bound to template strand with the two arms connected by a bridge containing seven base pairs. **f**. LNA389 (upper half of the Z-shaped LNA) and its corresponding target site **g.** LNA390 (lower half of the Z-shaped LNA) and its corresponding target site **h.** 27-mer LNA7472 and its corresponding target site **i.** 27-mer LNA7473 and its corresponding target site. Capital Letters, LNA; Small Letters, DNA; Cy-3, Cy-5, labeled probes, Acr, intercalating 9-aminoacridine; Lys, Lysines for enhanced binding affinity; J, pseudoisocytosine and eg, ethylene glycol (8-amino-3,6-dioxaoctanoic acid) linker units.

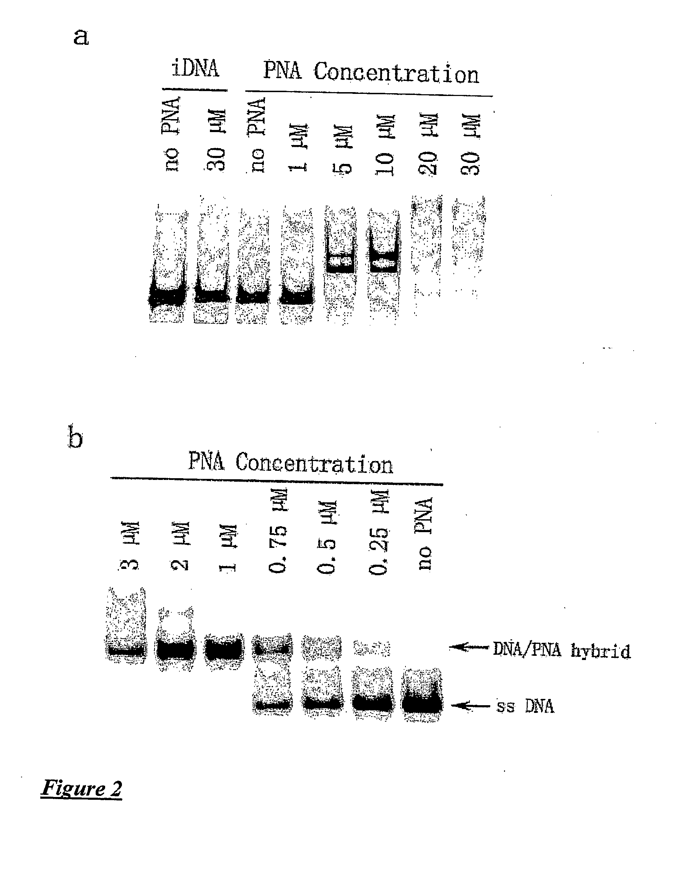

**Fig. 2** shows the analysis of the binding of PNA2582. **a,** A fixed concentration of duplex DNA oligos (1 microM) was incubated with increasing concentration of the PNA in 20 microl containing 20 mM sodium phosphate at 37°C overnight. iDNA, irrelevant control DNA lacking target sites. **b,** A fixed concentration of single stranded DNA (1 microM) was incubated with increasing concentration of the PNA in 20 microl containing 20 mM sodium phosphate at 37°C overnight. The reactions were analyzed by electrophoresis in a 15% native polyacrylamide gel.

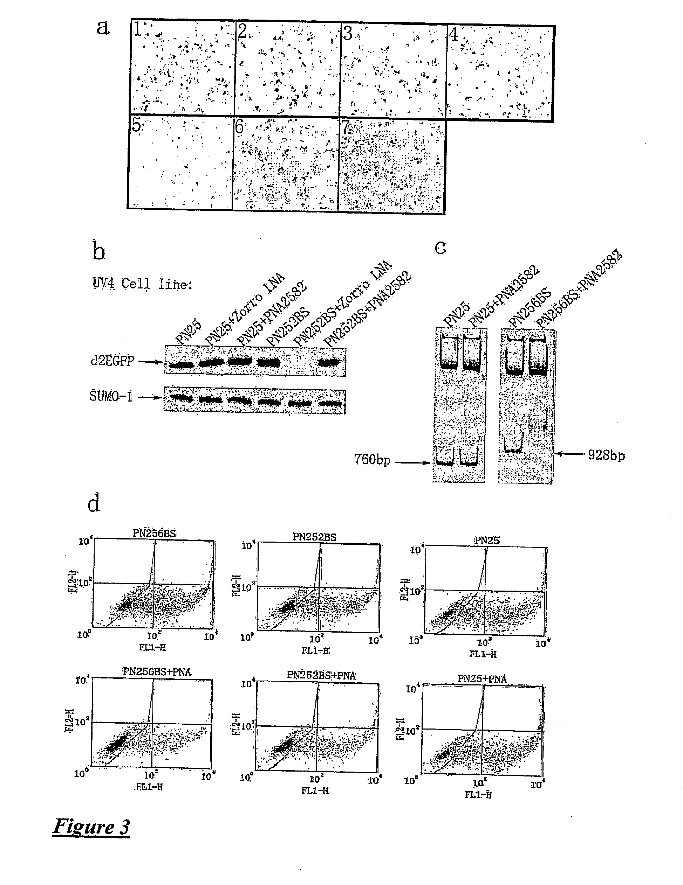

**Fig. 3** illustrates the effect of PNA triplex on destabilized green fluorescence protein expression. a, Plasmid PN25, with or without PNA binding sites, was pre-hybridized with PNA2582, and subsequently transfected into the U-2 OS cells. Intensity of the d2EGFP protein expression was monitored by fluorescence microscopic analysis after two days. 1, PN252BS without PNA, 2, PN252BS pre-hybridized with 20-fold molar excess of PNA2582, 3, PN256BS without PNA, 4, PN256BS pre-hybridized with 20-fold molar excess of PNA2582, 5, PN256BS pre-hybridized with 150-fold molar excess of PNA2582, 6, PN25 without PNA, 7, PN25 pre-hybridized with 150-fold molar excess of PNA2582. **b.** Plasmids PN25 and PN252BS were pre-hybridized with 20-fold molar excess of Z-shaped LNA oligomer and bisPNA2582, respectively, and then transfected into UV4 cells. After 48 hours, cells were harvested and d2EGFP protein expression was measured by western blot. **c.** Gel mobility experiment, digestion of plasmids PN25 and PN256BS pre-hybridized with or without 20-fold molar excess of PNA2582; left panel, no shift could be observed on the control plasmid PN25, right panel, approximately 100% target DNA was saturated and shifted on the plasmid PN256BS. **d.** Plasmids PN25, PN25-1BS, 2BS, 4BS, and 6BS were pre-hybridized with 150-fold molar excess of PNA2582, respectively, and then transfected into U-2 OS cells. After 48 hours, cells were harvested and d2EGFP expression was analyzed by FACS.

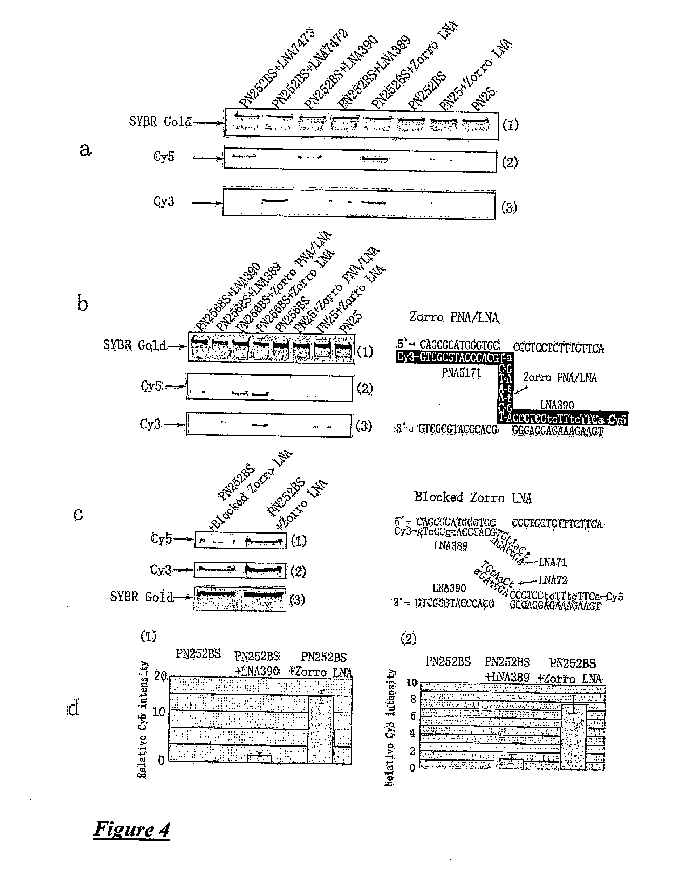

**Figure 4** shows the DNA duplex binding of Z-shaped LNA compared to other constructs. **a.** Plasmids PN25 and PN252BS(5 microg) were mixed with 10-fold molar excess of Z-shaped LNA, LNA389, LNA390, LNA7472 and LNA7473 respectively in 20 mM phosphate buffer (pH 6.8) at 37°C overnight, and analyzed on 4-20% polyacrylamide TBE gels. (1) Signals from supercoiled plasmid stained with SYBR Gold. (2) Signals from LNA labeled by Cy-5 probe. (3) Signals from LNA labeled by Cy-3 probe. **b.** Plasmids PN25 and PN256BS(3 microg) were mixed with 10-fold molar excess of Z-shaped LNA, LNA389, LNA390, mixed PNA5171/LNA390 respectively in 20 mM phosphate buffer (pH 6.8) and incubated at 37°C overnight. (1) Signals from supercoiled plasmid stained with SYBR Gold. (2) Signals from LNA labeled by Cy-5 probe. (3) Signals from LNA labeled by Cy-3 probe. Right panel, schematic representation of Z-shaped mixed PNA5171/LNA390. c. Plasmid PN252BS (3 microg) was mixed with 10-fold molar excess of Z-shaped LNA, Blocked Z-LNA (the bridge connecting the two LNA oligomers was blocked by complementary LNA71 and LNA72 hybridization prior to mixing the two Z-LNA arms), respectively in 20 mM phosphate buffer (pH 6.8) and incubated at 37°C overnight. (1) Signals from LNA labeled by Cy-5 probe. (2) Signals from LNA labeled by Cy-3 probe. (3) Signals from supercoiled plasmid stained with SYBR Gold. Right panel, schematic representation of Blocked Z-LNA. **d.** Densitometer analysis of different LNA constructs in binding efficiency. (1) Cy-5 intensity. (2). Cy-3 intensity.

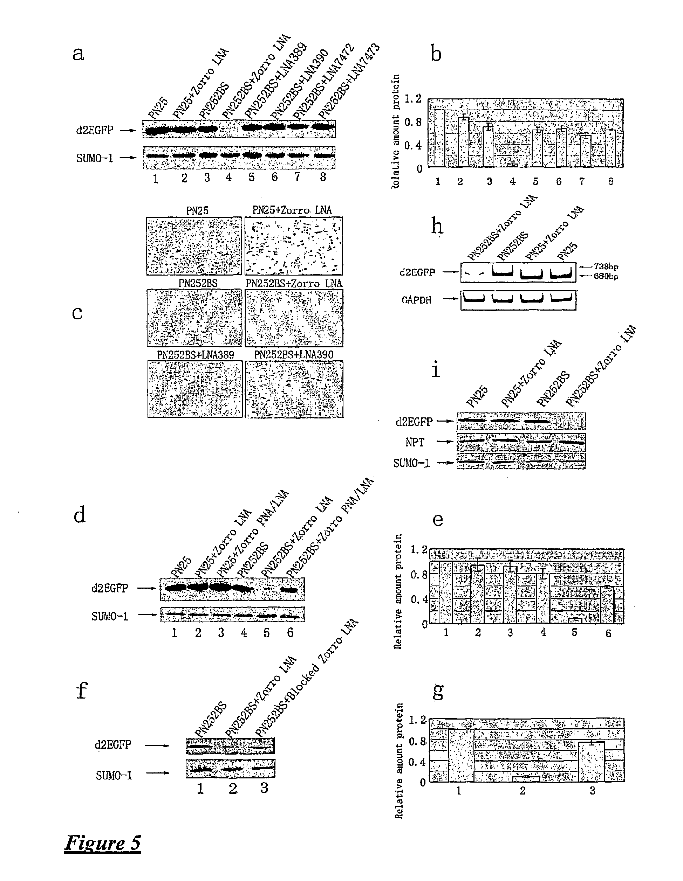

**Fig. 5** shows the inhibition of destabilized EGFP expression caused by Z-shaped LNA a, Plasmids PN25 and PN252BS were pre-hybridized with 10-fold molar excess of Z-shaped LNA oligomer and control LNA oligomers LNA389, LNA390, LNA7472, LNA7473, respectively, and subsequently transfected into U-2 OS. After 48 hours, cells were harvested and d2EGFP protein expression was measured by western blot. **b.** Densitometer analysis of inhibition in d2EGFP protein expression. **c.** Imaging of cells transfected as described in **a. d.** Plasmids PN25 and PN252BS were pre-hybridized with Z-shaped LNA and mixed Z-PNA/LNA, respectively, and subsequently transfected into U-2 OS. After 48 hours, cells were harvested and a d2EGFP protein expression was measured by western blot. **e.** Densitometer analysis of inhibition in d2EGFP expression. **f**. Plasmid PN252BS was pre-hybridized with Z-shaped LNA and Blocked Z-LNA (see figure 4 legend), respectively, and subsequently transfected into U-2 OS. After 48 hours, cells were harvested and a d2EGFP protein expression was measured by western blot. **g.** Densitometer analysis of inhibition in d2EGFP expression. **h.** d2EGFP mRNA level was determined by RT-PCR. Cellular transfection was as described in **a. i.** Plasmids PN25 and PN252BS were pre-hybridized with 20-fold molar excess of Z-shaped LNA, respectively, and then transfected into U2OS cells. After 48 hours, cells were harvested and d2EGFP and NPT protein expression was measured by western blot.

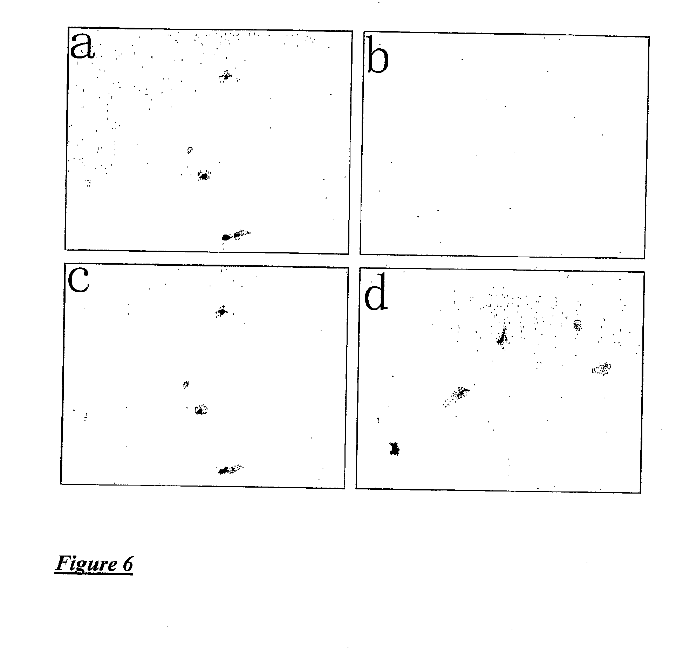

**Fig. 6** shows a Z-shaped LNA binding-mediated block of d2EGFP gene expression following micro-injection. 16 hours after intra-nuclear injection of LNA-bound PN252BS plasmid and mock-treated plasmid, d2EGFP gene expression was determined. In all cases, control plasmid, pDsRed2-N1, was co-injected. The d2EGFP fluorescence is shown in the top panels and DsRed2 fluorescence is shown in the bottom panels. **a** and **c** NIH-3T3 cells injected with PN252BS plasmid DNA alone. **b** and **d** NIH-3T3 injected with Z-shaped LNA-bound PN252BS.

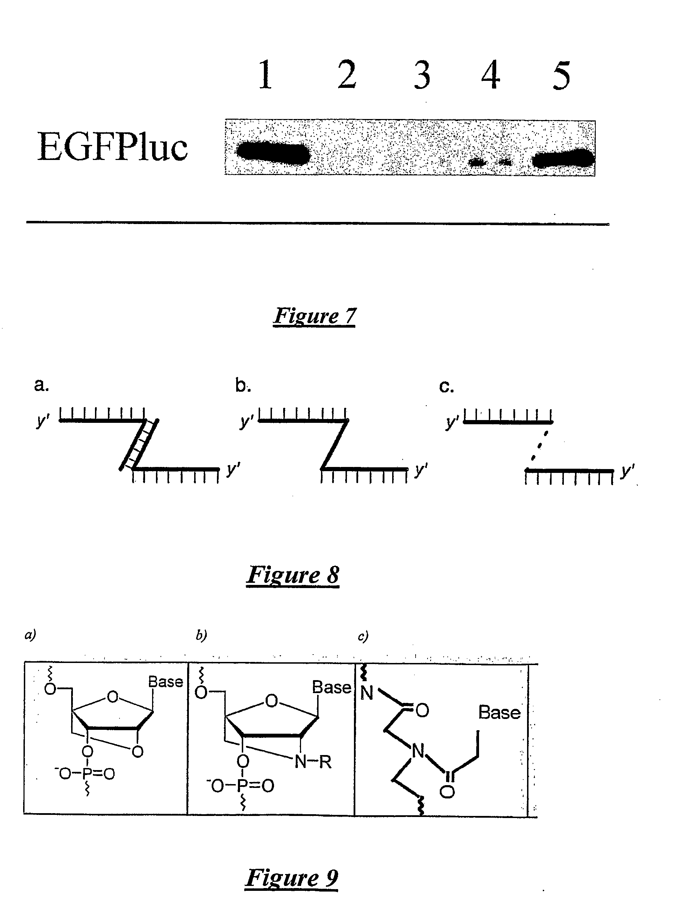

**Fig. 7** shows down-regulation of EGFP protein another promoter by blocking RNA polymerase III, which transcribes U6 promoter genes. Lane 1, only transfected with EGFPluc plasmid. Lane 2, co-transfected with EGFPluc and U6-shluc plasmid (0BS) (1:4). Lane 3, co-transfected with EGFPluc and Zorro LNA pre-hybridized-U6-shluc plasmid (0BS) (1:4). Lane 4, co-transfected with EGFPluc and U6-shluc plasmid (2BS) (1:4) Lane 5, co-transfected with EGFPluc and Zorro LNA pre-hybridized-U6-shluc plasmid (2BS) (1:4).

**Fig. 8** is a schematic presentation of the inventive Z-shaped construct formed through a) Watson-Crick like base pairing b) covalent linkage c) non-covalent linkage. The direction of the oligonucleotides is anti-parallel, y' is either 5' or 3'.

**Fig. 9** illustrates alternative chemical composition of the LNAs - either North or South ribose ring. a) oxy-LNA b) amino-LNA c) PNA.

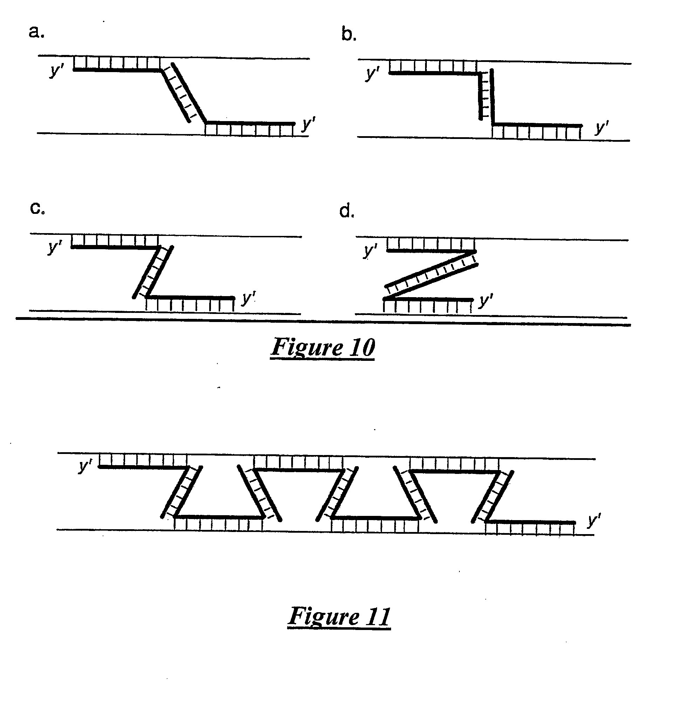

**Fig. 10** shows how, in an embodiment of the invention, both oligonucleotides are hybridized to the double stranded DNA in an anti-parallel trans way through Watson-Crick like base pairing.

**Fig. 11** illustrates an embodiment where a construct comprises multiple Z-shaped elements of extended Z-shaped elements.

### Description

The aim of the construct according to the present invention is to stabilize the hybridization between double stranded DNA (theoretically also RNA) through trans-hybridizing both strands. The inventors have utilized the inventive construct for transcription elongation arrest but other applications are possible; for example, but not limited to, translation and transcription and translation inhibition of eukaryotic (genomic) as well as bacterial or viral DNA/RNA, or as an anchoring entity as described in WO 00/15824 (PCT/SE99/00398) by the same applicant. In the description, examples and claims, the term "oligonucleotides" can be replaced by the term "oligomer".

The inventors have made available and tested a construct comprising of at least a first and a second oligonucleotide, wherein said first oligonucleotide is connected, at the 3' or 5' end respectively, to said second oligonucleotide 3' or 5' end respectively, the 3' end of said first oligonucleotide oriented towards the 5' end of said second oligonulecotide, and vice versa, through sequence specific Watson-Crick base pairing in an anti-parallel orientation. This construct is cost effective, flexible in combination and simple to synthesize.

The term "anti-parallel" means, in relation to the at least two oligonucleotides, that the two strands of DNA have opposite chemical polarity, or, stated another way, that their sugar-phosphate backbones run in opposite directions. Direction in nucleic acids is specified by referring to the carbons of the ribose ring in the sugar-phosphate backbone of DNA. 5' specifies the 5th carbon in the ribose ring, counting clockwise from the oxygen atom, and 3' specifies the 3rd carbon in the ring. Direction of, and in reference to, DNA molecules is then specified relative to these carbons. For example, transcription, the act of transcribing DNA to RNA for eventual expression, always occurs in the 5' to 3' direction. Nucleic acid polymerization cannot occur in the opposite direction, 3' to 5', because of the difference in chemical properties between the 5' methyl group and the 3' ring-carbon with an attached hydroxyl group.

According to another aspect, the oligonucleotides are chosen among DNA, RNA, analogs thereof or any mixture thereof, and wherein the composition of the oligonucleotides can be chosen independently for the different oligonucleotides. Preferably the DNA and/or RNA oligonucleotides have at least one inclusion of LNA.

According to the invention, the DNA and/or RNA oligonucleotides consist of LNA and/or PNA. Amino-LNA is not commercially available yet but due to its neutral charge, this could be a preferred choice. The R side chain could be anything; the simplest form would be hydrogen. Ordinary Oxy-LNA was used in the preliminary experiments. For strand invasion into double stranded DNA, South (DNA like) configuration of the ribose ring should be favored both for LNA and amino-LNA. Only the North configuration was however available.

According to yet another embodiment said construct is hybridized to double stranded DNA in a trans fashion. The oligonucleotide target sequence is preferably in close proximity, spaced by less than 5 bases. Alternatively, the oligonucleotide target sequence is adjacent. Preferably, the oligonucleotide target sequence is partly overlapping, most preferably by 1 to 8 bases. This should give the best stability of the hybrids without introducing self-hybridization complications of the intended DNA binding region. In the preliminary experiments, the present inventors used a one base overlap.

Many progenitor cells, including stem cells for various tissues, have been characterized over the last years. Embryonic stem (ES) cells are considered to be the most immature form of cells and have the capacity to differentiate into all other cell lineages. Also adult forms of stem cells exist. This differentiation process is regulated through a number of transcriptional activators and inhibitors. The Z-shaped construct could be used as a novel synthetic regulator of these processes. In one non-limiting example cultured ES-cells (or adult stem cells) could be made to differentiate into a particular cell lineage through the addition of one or more Z-shaped constructs. The use of the Z-shaped construct would be transient, since once a particular cell stage has developed it is not any longer necessary to add agents inducing differentiation. Such cells could be used for regenerative therapy in humans or in other species.

The inventors however also contemplate a construct, where the oligonucleotide target sequence is substantially or totally overlapping and complete self-hybridization of the construct is prohibited by the use of modified bases and/or modified backbone.

Also comprised in the invention is a construct as described above, wherein additional conjugates are added internally or at either end of the any of the oligonucleotides. Said additional conjugates are chosen among oligonucleotides, peptides, proteins, labels such as fluorescent or isotope labels, biologically active or inert molecules.

### Examples

### Methods

### Cell culture

The U-2 OS, COS-7, NIH-3T3 cells were obtained from the American Type Culture Collection (ATCC, HTB-96, Rockville, MD) and the UV4 cell line was obtained from Dr. Thomas Helleday (Stockholm University, Sweden). These cells were cultured in Dulbecco's modified Eagle's medium (DMEM) supplemented with 10% (v/v) heat-inactivated fetal calf serum and 100microg/ml of penicillin-streptomycin (Invitrogen, Stockholm, Sweden). Cells were cultured at 37 °C in a humidified 5% CO2 incubator.

### Plasmid constructs

Plasmids with 1, 2, 4 or 6 binding sites (BS), PN251BS, PN252BS, PN254BS and PN256BS were constructed by standard molecular cloning procedures. DNA fragments were inserted into *Bgl*II and *Avr*II sites of the PN25 plasmid, harboring a destabilized enhanced green fluorescent protein (d2EGFP) reporter driven by the human cytomegalovirus (CMV) early promoter/enhancer elements(20). The original plasmid was obtained from Dr. Piruz Nahreini (University of Colorado, CO).

### Transfection

Cells were seeded in six-well plates at a density of 1×10⁶ cells per well and allowed to attach for 18 hours prior to transfection. The plasmids PN252BS and PN256BS (1microg) were incubated with 10-fold molar excess of LNA oligomers or 20-fold or 150-folk molar excess of bisPNA (2.5microM) in 20 mM phosphate buffer (pH 6.8) at 37°C overnight. Fugene 6 Reagent (Roche Molecular Biochemicals, Stockholm, Sweden) was used to deliver the PNA/LNA-bound plasmid DNA. Transfection solution was prepared according to the manufacturer's protocol with serum-free medium. Cells were harvested for analysis of protein expression 48 or 72 hours after transfection. In co-transfection experiments, the plasmid PN256BS was mixed with increasing amount of PNA2582 (90-fold, 180-fold, 270-fold, 360-fold molar excess) immediately before transfection, and then the mixture of PNA and plasmid was transfected into U-2 OS cells by Fugene 6. Cells were harvested for analysis of protein expression 48 hours after transfection. All of the experiments were repeated at least three times. Values are presented as the mean of a triplicate ((+/-) SD) from a representative experiment.

### Oligonucleotides, PNA and LNA

Oligoilucleotides containing bisPNA and Z-shaped LNA binding sites (5'-CAGCGCATGGGTGCCCCTCCTCTTTCTTCA-3' and 5'-TGAAGAAAGAGGAGGGGCACCCATGCGCTG-3') and primers used in this study were synthesized by DNA Technology A/S (Aarhus, Denmark) or Cybergene AB (Huddinge, Sweden) and purified with Cartridge Purification. Peptide Nucleic Acid, PNA2582, shown in Figure 1 (Acr-Lys-Lys-CCCCTCCTCTTTCTT-(eg1)3-TTJTTTJTJJ-Lys-NH2, Acr, intercalating 9-aminoacridine; Lys, Lysine) was obtained from Professor Peter Nielsen (University of Copenhagen, Denmark). PNA was synthesized as previously described(21). PNA5171, shown in Figure 4b (n-OTCAATCTGCACCCATGCGCTGCTG-n) was purchased from BioSynthesis Inc. (Lewisville, TX), purified with HPLC. A series of Cy-3 or Cy-5 end-labeled, HPLC-purified Locked Nucleic Acids, shown in Figure 1 and Figure 4c, were purchased from Proligo SAS (Paris, France) (Fig. 1).

### PNA binding assay

PNA binding to the target duplex was measured by using a gel mobility shift assay. Two complementary 59-mers containing the PNA binding site were synthesized. The duplex DNA was prepared by mixing both 59-mers at a ratio of 1:1 in TE buffer (10 mM Tris/1mM EDTA, pH 8.0) and incubating the solution at 95°C for 5 minutes and then cooling it down to room temperature slowly. A fixed concentration of duplex DNA or single stranded DNA strand of 59-mers containing the PNA binding site (1microM) were incubated with increasing concentration of the PNA in 20microL a solution containing 20 mM sodium phosphate at 37°C overnight, respectively. The reactions were analyzed by electrophoresis in a 15% native polyacrylamide gel.

### LNA binding assay

Cy-3/Cy-5 labeled LNAs binding to the target sites of plasmid was detected using Molecular Imager FX equipment (Bio-Rad Laboratories, Sundbyberg, Sweden). 1microg of plasmid DNA PN25, PN252BS or PN256BS were mixed with 10-fold molar excess of LNA oligomers or mixed LNA/PNA oligomer in 20 mM phosphate buffer (pH 6.8) and incubated at 37°C overnight, respectively. This reaction was analyzed by electrophoresis in Novex® 4-20% polyacrylamide TBE gels (Invitrogen).

### RT-PCR

Total RNA was prepared using the Qiagen RNA/DNA mini Kit (Qiagen, Stockholm, Sweden), and RT-PCR was performed using the Qiagen Onestep RT-PCR Kit (Qiagen) and gene specific primers according to the manufacturer's protocol. Forward and reverse primers for d2EGFP were 5'-TCAGATCGCCTGGAGACG-3' and 5'-TGTTCTGCTGGTAGTGGTC-3', respectively. GAPDH primers were 5'-GGGTGTGGGCAAGGTCATCC-3' and 5'-TCCACCACCCTGTTGCTGTA-3', respectively (22). RT-PCR products were analyzed on Novex® 4-20% polyacrylamide TBE gels (Invitrogen). The d2EGFP primers amplified a fragment of 680bp (PN25) and 738bp (PN252BS), respectively. To the detection of d2EGFP and GAPDH transcripts in the exponential phase of amplification, initial experiments were performed to optimize assay condition (i.e. number of cycles, primer concentration and amount of RNA template).

### Western blot analysis

Cells were lysed in boiling lysis buffer (2% SDS, 10 mM Tris-HCl, pH 6.8), and protein lysate was fractionated by 4-20% Novex® SDS-PAGE gels (Invitrogen) and transferred to nitrocellulose membranes (Advantec MFS, Dublin, CA). The nitrocellulose membrane was incubated with blocking buffer (5% dried milk in PBS and 0.1% Tween-20), probed with a 1:5000 dilution of rabbit anti-GFP antibody (BD Biosciences, Stockholm, Sweden) followed by goat antirabbit IgG conjugated to horseradish peroxidase (1:2000 dilution). Immune complexes were detected with the Supersignal® West Femto Chemiluminescence Western Blotting detection system (Pierce, Rockford, IL). Anti-SUMO-1 antibody (Invitrogen) was used to detect a heavily 90 kDa sumolated protein as internal control. Anti-Neomycin Phosphotransferase II (Upstate, Charlottesville, VA) was applied to detect Neomycin Phosphotransferase.

### Microinjection and immunofluorescence

The plasmid PN252BS (2microg) was incubated with or without 10-fold molar excess of Z-shaped LNA (2.5microM) in 20 mM phosphate buffer (pH 6.8) at 37 °C overnight. The LNA-bound plasmid and mock-treated plasmid were adjusted to a concentration of 0.1microg/microl. NIH-3T3 cells were grown to 50% confluence on 22mm cover-slips and were co-injected with 0.25microg/microl pDsRed2-N1 (Invitrogen) and 0.1microg/microl LNA-bound plasmid or mock-treated plasmid, respectively, into the nucleus using an Eppendorf 5246 Microinjector. 16 hours after microinjection, cells were visualized by a Carl Zeiss fluorescence microscope.

### FACS analysis

Cells were pre-seeded in six wells, transfected as described in the transfection section. 48 hours after transfection, cells were harvested and analyzed by FACSCalibur flow cytometer (BD Biosciences).

### Results

### Efficient binding of PNA to a linear DNA target sequence

To examine PNA binding to a homopurine target sequence, a gel mobility shift assay was applied. Synthetic DNA oligomers of 59 bp with a single, central anchor site were used as duplex targets for PNA binding (Fig. 1a). The anchor site consists of 15 nucleotides complementary to the extended bisPNA, which hybridize through Watson-Crick base pairing, whilst 10 nucleotides bind in the Hoogsteen mode. In order to optimize hybridization the present inventors used a tail-clamp PNA, designated PNA2582, containing 3 lysine residues, which increases DNA binding by charge interactions, as well as an intercalating aminoacridine moiety for maximal activity. Addition of aminoacridine has been reported to enhance DNA binding affinity by 20 to 150-fold (23). In PNA2582 cytosine residues were substituted with pseudoisocytosines, also referred to as J-bases, in order to further promote hybridization. The samples were analyzed for binding by gel mobility shift assay using native PAGE (Fig. 2a). Increasing amounts of PNA were incubated with fixed concentrations of duplex target DNA. As the concentration of the specific PNA, PNA2582, was augmented, the proportion of the target duplex bound increased, as manifest by the reduced mobility of the PNA-DNA complex. At a concentration of 5microM (PNA:DNA ratio 5:1) essentially all of the 59-mer target oligos were shifted yielding two fragments with slower mobility. The exact composition of these two species is not exactly known, but a modest degree of stacking may exist on the lower-mobility form. From a PNA concentration of 20microM, a blurred pattern was observed with the 59-mer target oligos displaying further reduced mobility. This result may be due to the formation of big complexes between DNA and PNA, perhaps due to stacking(24). Very weak binding was detected to the control doublestranded 59-mer DNA oligos lacking target sequences (iDNA), only visible at the highest PNA concentration of 30microM (Fig. 2a). In order to demonstrate that the capability of PNA binding to DNA exerts a linear relationship with the ratio of PNA:DNA, 59 bp single-stranded DNA oligos (1microM) with a single central anchor site was incubated with increasing amount of PNA. The samples were analyzed for binding by gel mobility shift assay using native PAGE (Fig. 2b). These results suggest that PNA can induce effective strand invasion and form highly stable triplexes at modest PNA to DNA target site ratios, as manifested by the binding to a linear DNA duplex, and that PNA invasion into the DNA duplex is strictly concentration dependent. Under these conditions, which were mainly used to verify sequence-specific binding of the PNA, the PNA:DNA ratio was modest, at the maximum 30:1. The present inventors believe that formation of complexes was favored by the relatively high, final concentration applied when hybridizing to the DNA oligonucleotide together with the strong binding efficacy of this optimized PNA oligonucleotide.

### Effect of bisPNA. triplex on destabilized green fluorescence protein expression in mammalian cells

To determine if the tail-clamp bisPNA could arrest transcription elongation in mammalian cells, as was previously reported for *in vitro* transcription (16, 17) PNA was bound to anchor sites generated in EGFP reporter plasmids and transfected into cultured human U-2 OS osteosarcoma cells. Due to the 8 high stability of EGFP protein, the present inventors used a destabilized form, d2EGFP, with a half-life of only 2 hours(25). Plasmids were generated to contain a single, or multiples, of 2, 4 or 6 homopurine stretches of 15 nucleotides each, introduced at *Bgl*II and *Avr*II restriction sites located between a CMV promoter and the d2EGFP reporter gene. The homopurine stretches served as anchor sites for the aminoacridine-containing, tail-clamp PNA2582. The constructed PN25 and PN252BS plasmids, which are depicted in Fig. 1 panel b and c, as well as the PN256BS plasmids (1microg), were mixed with 20-fold molar excess of PNA2582 per binding site and incubated in 20 mM phosphate buffer at 37°C overnight to form the PNA-DNA-PNA triplex structures (Fig. 1d). PNA-bound plasmid and mock-treated plasmid were transfected into the U-2 OS cells using Fugene 6. The intensity of the d2EGFP protein expression was monitored by fluorescent microscopic analysis after two days of culture (Fig. 3a). No decrease in GFP expression was detected in U-2 OS cell transfected with plasmids containing 1, 2 or 6 anchor sites, that were pretreated with 20-fold molar excess of PNA2582 (Fig. 3a and data not shown). Interestingly, the lack of an effect was observed in spite of that the gel mobility shifts of restriction enzyme (*Hind*III) digested plasmids proved that essentially all target sites on the plasmid were occupied by PNA2582 (Fig. 3c, right panel and data not shown). In contrast, significant down-regulation in d2EGFP protein expression was observed in U-2 OS cells transfected with the PN256BS plasmid, which was pretreated with 150-fold molar excess of PNA2582 per binding site (Fig. 3a, panel 5). Since the molar ratio of PNA:plasmid in this case was 900:1, this phenomenon is likely due to formation of big complexes around the multiple binding sites caused by the presence of a very high excess of PNA. In order to investigate the influence of 9 the number of binding sites for the inhibition of gene expression, the present inventors studied plasmids with 1, 2, 4 or 6 PNA anchor sites to observe the effect of the tail-clamp PNA, PNA2582. All plasmids were pre-hybridized with 150-fold molar excess of PNA2582 per binding site, and then the PNA-bound plasmids were transfected into U-2 OS cells. After 48 hours of culture, the intensity of the d2EGFP protein expression was monitored by FACS analysis (Fig. 3d). Densitometer measurements revealed that the plasmid carrying 6 binding sites was inhibited by 85% in d2EGFP expression. As the number of binding sites decreased, the degree of inhibition became reduced (4BS plasmid 65% inhibition, 2BS plasmid 40% inhibition, 1BS plasmid ≤20% inhibition; no decrease in expression of d2EGFP observed on control plasmid, PN25) . Subsequently, the present inventors co-transfected PN256BS plasmid with increasing amounts of PNA2582. After 48 hours of culture, the d2EGFP protein expression was monitored by western blot analysis . Densitometer measurements revealed that a 360-fold molar excess of PNA caused maximal inhibition of d2EGFP (90%), whereas as the amount of PNA decreased, the efficiency of inhibition was reduced

. Similar results were seen in NIH-3T3, COS-7 cell lines as well (data not shown). In order to define the specific effect of PNA on transcription, the present inventors also measured the expression level of the Neomycin resistance gene located in cis and driven by the LTR promoter (Fig.1c). This analysis demonstrated that PNA did not affect transcription of Neomycin resistance gene

### Effect of binding of Z-shaped LNA to DNA

Because PNA did not block transcription of reporter genes when target site binding was saturated at PNA:DNA ratios of 20:1, the present inventors set out to analyze the effect of another nucleic acid analog, namely LNA. Previous experience using LNA had taught the inventors that, compared to bisPNA, LNA binds less well to doublestranded DNA (26) (and unpublished data). For this reason, a novel type of construct, which simultaneously binds both strands of a DNA duplex (Fig. 1e) was initially tested. Due to the shape of this oligomer, forming a Z-like structure, and to avoid any confusion with the left-handed Z-form of DNA, the inventors designated these constructs "Z-shaped LNA". Moreover, it is the experience of the present inventors that hybridization of LNA to DNA causes less of retardation in gel mobility shift assays as compared to the non-charged PNA. For this reason, all of the LNA oligomers, used in this study, were end-labeled with Cy-3 or Cy-5 fluorophores to simplify their detection. Highly purified supercoiled plasmid PN25 and PN252BS (5microg) were mixed with 10-fold molar excess of Z-shaped LNA, formed by the 14-mer LNA389 together with the 16-mer LNA390. As controls, 27-mer linear LNAs, designated LNA7472 and LNA7473 (Fig. 1h and i) were included. LNA bound supercoiled plasmids and control plasmids were run on 4-20% polyacrylamide TBE gels (Fig. 4a). Supercoiled plasmid was stained by SYBR Gold (Fig. 4a, panel 1). Exposure for Cy-5 revealed that binding induced by Z-shaped LNA was much more powerful than that induced by one of the linear Z-arms, LNA390, (16-mer, lower half of Z-shaped LNA; Fig. 1g), whereas on control plasmid PN25, lacking binding sites, only very weak unspecific binding by Z-shaped LNA was seen (Fig.4a, panel 2). Exposure for Cy-3 revealed that binding induced by Z-shaped LNA was also stronger than that induced by the other arm, LNA389, (14-mer, upper half of Z-shaped LNA; Fig. 1f). Likewise, only very weak unspecific binding of Z-shaped LNA to the control plasmid was seen when Cy-3 was analyzed (Fig. 4a, panel 3). To distinguish between the possibilities that the enhanced signal in duplex DNA binding by Z-shaped LNA is due to the characteristic Z-structure or caused by the longer hybridization region, to which both halves of the "Z" contribute, two linear 27-mer control LNA oligomers were used, 11 antisense LNA7472 and sense LNA7473, respectively (Fig. 1h and i). It was found that binding of these extended, single-stranded LNA 27-mers, is also very effective. Although, as seen in Fig. 4a, the inventors noticed that antisense 27-mer linear LNA7472 gave a stronger signal, as compared to sense 27- mer linear LNA7473 signal (Fig.4a, panels 2 and 3), this is most likely because the LNA7473 oligo contained a lower amount of fluorophore label (data not shown).

### Binding of Z-shaped PNA-LNA hybrids to a DNA duplex

In order to compare the hybridizing ability of Z-shaped LNA to that of similarly shaped PNA,the same two arms were synthesized based upon PNA chemistry. To promote solubility, 2 asparagine residues were added to the N-terminus (5' end). Asparagine rather than lysine was used in order to avoid the influence of charge interactions, which may favor hybridization to the plasmid thereby violating the comparison with LNA, which is negatively charged. Conversely, the putative interaction between the positively charged lysine residues and the negatively charged LNA may instead impair its strand-invading capacity. However, in spite of that each PNA-arm was soluble, when mixed to allow for the bridge to form by hybridization, it was observed that the solubility decreased considerably, a phenomenon not infrequently seen when using long PNA sequences(27). The present inventors therefore investigated a hybrid form of LNA-PNA Z-shaped construct for binding in which one arm (LNA389) in Z-shaped LNA was substituted by a PNA arm (PNA5171) with identical sequence (Fig. 4b, right panel). The analysis showed that the Cy-5 signal becomes much weaker, if one arm of Z-shaped LNA (LNA389) was replaced by PNA5171 (Fig. 4b, panel 2). Likewise, Cy-3 exposure revealed that binding induced by Z-shaped LNA was much more powerful than that induced by a Z-hybrid with one PNA and one LNA arm (Fig. 4b, panel 3). This result could be because the melting temperature of DNA/PNA hybrids is lower than that of DNA/LNA(28). To understand the importance of the bridge structure between the two LNA arms for binding, two 7-mer LNA oligomers (LNA71 and LNA72; Fig. 4c, right panel) were used to block the mutual binding of two LNA arms of Z-shaped LNA. This experiment showed that the Cy-5 signal became much weaker, if the bridge structure of Z-shaped LNA was blocked (Fig.4c, panel 1). Similarly, Cy-3 exposure showed that the binding induced by an intact, Z-shaped LNA was more efficient than that induced following abrogation of bridge formation in the Z-LNA (Fig. 4c, panel 2). In summary, the binding activity to the DNA duplex of a 14-mer, or a 16-mer, linear LNA is quite limited, as compared to Z-shaped LNA or 27-mer linear LNAs. Densitometer measurements revealed that binding induced by Z-shaped LNA was 10-fold more efficient than that induced by LNA390 and 7-fold more efficient than that induced by LNA389 (Fig. 4d). Moreover, if one arm of Z-shaped LNA was substituted by a PNA oligomer, the effect of binding to a DNA duplex decreased significantly. Thirdly, if the linkage of the bridge structure between the two LNAs of Z-shaped LNA was blocked, binding was decreased significantly as well.

### Inhibition of destabilized green fluorescence protein caused by Z-shaped LNA

In order to investigate if LNA could serve as an anti-gene reagent to block gene transcription in a sequence-specific way, the present inventors further studied the effect of the novel, Z-shaped LNA construct on the expression of d2EGFP protein in human osteosarcoma cells. The PN252BS plasmid (1microg) with 13 two binding sites was pre-hybridized with 10-fold molar excess of Z-shaped LNA oligomer or with control LNA oligomers LNA389, LNA390, LNA7472, LNA7473, respectively (Fig. 1f-i) in 20mM phosphate buffer at 37°C overnight. The Z-shaped, LNA-bound plasmid, mock-treated plasmid and other control samples were transfected into pre-seeded U-2 OS. After 48 hours, cells were harvested and d2EGFP protein expression was measured by western blot of cellular lysates (Fig. 5a). Densitometer measurements revealed a ≥90 % specific inhibition of d2EGFP protein expression in the Z-shaped LNA-bound plasmid. No significant decrease in d2EGFP protein expression was seen using mock-treated plasmid or any of the control LNA oligomers (Fig. 5b). The same results were achieved when analyzing transfected cells by a fluorescence microscope (Fig. 5c). In order to define the specific effect of PNA on transcription, the inventors also measured the expression level of Neomycin resistant gene, which was driven by LTR promoter (Fig.1c). It demonstrated that Z-shaped LNA did not affect transcription of Neomycin resistant gene (Fig. 5i). In addition, the level of d2EGFP mRNA was assessed by RT-PCR. The mRNA level was equally reduced by ≥90 % in cells transfected by Z-shaped LNA bound PN252BS plasmid, whereas there was no significant decrease in level of d2EGFP mRNA detected in cells transfected by PN25 control plasmid treated with Z-shaped LNA. The level of GAPDH mRNA indicated equal RNA loading (Fig. 5h). Similar results were seen in the fibroblast cell line, NIH-3T3 (data not shown). The inventors also compared the ability of Z-shaped LNA with that of a Z-PNA-LNA hybrid (Fig. 4b, right panel) for inhibition of gene transcription in osteosarcoma cells. The plasmids PN25 and PN252BS were pre-hybridized with Z-LNA or Z-PNA-LNA hybrids, respectively, and subsequently transfected into U-2 OS cells. After 48 hours, cells were harvested and analyzed by western blot (Fig. 5d). Densitometer measurements revealed that pronounced decrease in expression 14 of d2EGFP protein was induced by Z-shaped LNA, whereas Z-PNA-LNA hybrids only induced 30% decrease in expression of d2EGFP (Fig. 5e). No significant decrease in protein expression was observed on the mock plasmid. Comparison of unmodified, Z-shaped LNA to the blocked version, in which the formation of a bridge was abrogated (Fig. 4c, right panel), for inhibition of gene transcription in osteosarcoma cells was carried out as well. Densitometer measurements revealed a pronounced decrease in expression of d2EGFP protein by Z-shaped LNA, whereas Z-LNA in which the formation of a bridge was inhibited only induced 20-30% decrease in expression of d2EGFP (Fig. 5g), in concordance with the results from the binding analysis. In order to rule out the possibility that cellular uptake or nuclear delivery could be affected by binding of Z-shaped LNA, the inventors performed intra-nuclear microinjection of pre-hybridized PN252BS with Z-shaped LNA into NIH-3T3 cells. Sixteen hours after injection of LNA-bound PN252BS plasmid and mock-treated plasmid, d2EGFP gene expression was determined (Fig.6). The intensity of d2EGFP gene expression was significantly decreased in the cells which were injected with Z-shaped LNA-bound plasmid (Fig.6b), comparing with the intensity of d2EGFP expression in the cells which were injected with plasmid DNA alone (Fig.6a). In all cases, control plasmid, pDsRed2-N1, was co-injected as a marker (Fig.6c and 6d).

### Inhibition of polymerase 3 transcribed green fluorescence protein caused by Z-shaped LNA

To investigate if LNA could serve as an anti-gene reagent for other polymerases, an U6 promoter was used for the transcription of EGFP. A U6 promoter is transcribed by RNA polymerase III. Incubate the LNA with plasmid at 37°C overnight in pH 6.8, 20 mM phosphate buffer. Transfection was performed after the incubation with Fugene 6 reagent (Roche). After 48 hours, the cells were harvested to detect the expression of EGFPluc fusion protein by Western blot as described earlier.

### DNA repair is not removing DNA-bound PNA

A possible explanation for the failure of saturated PNA binding from blocking transcription in living cells is that the PNA is removed during transcription and another possibility is that the DNA repair machinery recognizes the altered structure and excises this portion of the plasmid. To investigate if PNA forming triplexes could activate DNA repair mechanism in the cells, PNA bound reporter gene plamids were transfected into UV4 cells, which are defective in nucleotide excision repair(29). After 48 hours, cells were harvested and d2EGFP protein expression was measured by western blot of cellular lysates (Fig. 3b). No decrease in d2EGFP protein expression was detected using plasmids pre-hybridized with 20-fold molar excess of bisPNA 2582, whereas gene silencing was readily seen in the sample using the Z-shaped LNA-bound plasmid. According to gel shifts analysis, 20-fold molar excess of PNA was enough to saturate target sites on the plasmid (Fig. 3c). Thus, no inhibition of protein expression could be observed in several mammalian cell lines of different origins, including UV4 cells, which are defective in nucleotide excision repair. Although inhibition could be seen in cells transfected with PN256BS when pre-hybridized in 150-fold molar excess of PNA, this phenomenon could be because supra-molecular complexes are formed around the multiple binding sites in this plasmid.

### References

1. Koshkin, A. A. & Wengel, J. (1998) J Org Chem 63,2778-2781.
2. Braasch, D. A. & Corey, D. R. (2001) Chem Biol 8, 1-7.
3. Braasch, D. A., Liu, Y. & Corey, D. R. (2002) Nucleic Acids Res 30, 5160-7.
4. Grunweller, A., Wyszko, E., Bieber, B., Jahnel, R., Erdmann, V. A. & Kurreck, J. (2003) Nucleic Acids Res 31,3185-93.
5. Frieden, M., Christensen, S. M., Mikkelsen, N. D., Rosenbohm, C., Thrue, C. A., Westergaard, M., Hansen, H. F., Orum, H. & Koch, T. (2003) Nucleic Acids Res 31, 6365-72.
6. Crinelli, R., Bianchi, M., Gentilini, L., Palma, L., Sorensen, M. D., Bryld, T., Babu, R. B., Arar, K., Wengel, J. & Magnani, M. (2004) Nucleic Acids Res 32, 1874-85.
7. Schmidt, K. S., Borkowski, S., Kurreck, J., Stephens, A. W., Bald, R., Hecht, M., Friebe, M., Dinkelborg, L. & Erdmann, V. A. (2004) Nucleic Acids Res 32, 5757-65.
8. Vester, B., Lundberg, L. B., Sorensen, M. D., Babu, B. R., Douthwaite, S. & Wengel, J. (2002) J Am Chem Soc 124, 13682-3.
9. Parekh-Olmedo, H., Drury, M. & Kmiec, E. B. (2002) Chem Biol 9, 1073-84.
10. Nielsen, P. E., Egholm, M., Berg, R. H. & Buchardt, O. (1991) Science 254, 1497-500.
11. Egholm, M., Buchardt, O., Christensen, L., Behrens, C., Freier, S. M., Driver, D. A., Berg, R. H., Kim, S. K., Norden, B. & Nielsen, P. E. (1993) Nature 365, 566-8.
12. Hanvey, J. C., Peffer, N. J., Bisi, J. E., Thomson, S. A., Cadilla, R., Josey, J. A., Ricca, D. J., Hassman, C. F., Bonham, M. A., Au, K. G. & et al. (1992) Science 258, 1481-5.
13. Peffer, N. J., Hanvey, J. C., Bisi, J. E., Thomson, S. A., Hassman, C. F., Noble, S. A. & Babiss, L. E. (1993) Proc Natl Acad Sci USA 90, 10648-52.
14. Demidov, V. V., Yavnilovich, M. V., Belotserkovskii, B. P., Frank-Kamenetskii, M. D. & Nielsen, P. E. (1995) Proc Natl Acad Sci U S A 92, 2637-41.
15. Demidov, V. V., Potaman, V. N., Frank-Kamenetskii, M. D., Egholm, M., Buchard, O., Sonnichsen, S. H. & Nielsen, P. E. (1994) Biochem Pharmacol 48, 1310-3.
16. Bentin, T., Larsen, H. J. & Nielsen, P. E. (2003) Biochemistry 42, 13987-95.
17. Kaihatsu, K., Shah, R. H., Zhao, X. & Corey, D. R. (2003) Biochemistry 42, 13996-4003.
18. Nielsen, P. E., Egholm, M. & Buchardt, O. (1994) Gene 149, 139-45.
19. Mologni, L., Nielsen, P. E. & Gambacorti-Passerini, C. (1999) Biochem Biophys Res Commun 264, 537-43.
20. Andreatta, C., Nahreini, P., Hovland, A. R., Kumar, B., Edwards-Prasad, J. & Prasad, K. N. (2001) Biotechniques 30,656-60.
21. Christensen, L., Fitzpatrick, R., Gildea, B., Petersen, K. H., Hansen, H. F., Koch, T., Egholm,, M., Buchardt, O., Nielsen, P. E., Coull, J. & et al. (1995) J Pept Sci 1, 175-83.
22. Catapano, C. V., McGuffie, E. M., Pacheco, D. & Carbone, G. M. (2000) Biochemistry 39, 5126-38.
23. Bentin, T. & Nielsen, P. E. (2003) J Am Chem Soc 125, 6378-9.
24. Petersson, B., Nielsen, B.B., Rasmussen, H., Larsen, I.K., Gajhede, M., Nielsen, P.E. and Kastrup, J.S. (2005) J Am Chem Soc 127, 1424-1430.
25. Li, X., Zhao, X., Fang, Y., Jiang, X., Duong, T., Fan, C., Huang, C. C. & Kain, S. R. (1998) J Biol Chem 273, 34970-5.
26. Lundin, K. E., Hasan, M., Moreno, P. M., Tornquist, E., Oprea, I., Svahn, M. G., Simonson, E. O. & Smith, C. I. (2005) Biomol Eng 22, 185-92.
27. Nielsen, P. E. (2004) Mol Biotechnol 26, 233-48.
28. Ng, P. S. & Bergstrom, D. E. (2005) Nano Lett 5, 107-11.
29. Tebbs, R. S., Salazar, E. P. & Thompson, L. H. (2001) Environ Mol Mutagen 38, 111-7.

## Claims

1. A construct comprising at least a first and a second oligonucleotide, connected to one another, wherein the oligonucleotides are chosen among DNA, RNA, analogs thereof or any mixture thereof, and wherein the composition of the oligonucleotides can be chosen independently for the different oligonucleotides, **characterized in that** said first and second oligonucleotides consist of LNA and/or PNA and together have a structure as depicted in figure 10, said oligonucleotides are connected to one another through sequence specific Watson-Crick base pairing, and wherein said construct hybridizes through sequence specific Watson-Crick base pairing to double stranded DNA in a trans fashion invading the strand to affect transcription.

2. The construct according to claim 1, wherein in cases when more than two oligonucleotides are used, at least one is connected at both ends.

3. The construct according to claim 1, wherein the oligonucleotide target sequences on the double stranded DNA are in close proximity, spaced by less than 5 bases.

4. The construct according to claim 1, wherein the oligonucleotide target sequences are adjacent.

5. The construct according to claim 1, wherein the oligonucleotide target sequences are partly overlapping.

6. The construct according to claim 5, wherein the oligonucleotide target sequences are overlapping by 1 to 8 bases

7. The construct according to claim 5, wherein the oligonucleotide target sequences are substantially, or totally, overlapping and complete, or partial, but significant self-hybridization of the construct is prohibited by the use of modified bases and/or modified backbone.

8. The construct according to any one of the claims above, wherein additional conjugates are added internally or at either end of any of the oligonucleotides.

9. The construct according to claim 8, wherein said additional conjugates are chosen among oligonucleotides, peptides, proteins, labels such as fluorescent or isotope labels, biologically active or inert molecules.

10. The use of a construct according to any one of claims 1 - 9, for *in vitro* translation and transcription and translation inhibition of DNA/RNA by strand invasion of double stranded DNA.

11. The use according to claim 10, wherein the DNA or RNA is of eukaryotic (genomic), bacterial or viral origin.

12. The use according to claim 10, wherein the DNA or RNA is a tumor gene.

13. The use according to claim 10, wherein the DNA or RNA is a gene regulating the differentiation of cells in tissue cultures.

14. The use of a construct according to any one of claims 1 - 9, for the manufacture of a medicament for therapeutic silencing of gene expression.

15. The use according to claim 14, wherein the gene is a tumor gene.

16. An *in vitro* method for the translation and transcription inhibition of DNA or RNA by strand invasion of double stranded DNA, wherein a construct according to any one of claims 1- 9 is used.

17. The method according to claim 16, wherein the DNA or RNA is of eukaryotic (genomic), bacterial or viral origin.

## Patentansprüche

1. Konstrukt, umfassend mindestens ein erstes und ein zweites Oligonukleotid, die miteinander verbunden sind, wobei die Oligonukleotide ausgewählt sind aus DNA, RNA, Analogen davon oder einem Gemisch davon und wobei die Zusammensetzung der Oligonukleotide unabhängig für die verschiedenen Oligonukleotide ausgewählt werden kann, **dadurch gekennzeichnet, dass** die ersten und zweiten Oligonukleotide aus LNA und/oder PNA bestehen und zusammen eine Struktur aufweisen, wie sie in Figur 10 dargestellt ist, wobei die Oligonukleotide durch sequenzspezifische Watson-Crick-Basenpaarung miteinander verbunden sind und wobei das Konstrukt durch sequenzspezifische Watson-Crick-Basenpaarung in Trans-Form mit doppelsträngiger DNA hybridisiert, wobei es in den Strang eindringt, um die Transkription zu beeinflussen.

2. Konstrukt nach Anspruch 1, wobei in Fällen, wenn mehr als zwei Oligonukleotide verwendet werden, mindestens eines an beiden Enden verbunden ist.

3. Konstrukt nach Anspruch 1, wobei sich die Oligonukleotidzielsequenzen auf der doppelsträngigen DNA nahe beieinander befinden und durch weniger als 5 Basen getrennt sind.

4. Konstrukt nach Anspruch 1, wobei die Oligonukleotidzielsequenzen nebeneinander liegend sind.

5. Konstrukt nach Anspruch 1, wobei die Oligonukleotidzielsequenzen teilweise überlappend sind.

6. Konstrukt nach Anspruch 5, wobei die Oligonukleotidzielsequenzen um 1 bis 8 Basen überlappend sind.

7. Konstrukt nach Anspruch 5, wobei die Oligonukleotidzielsequenzen im Wesentlichen oder vollständig überlappend sind und die vollständige oder teilweise, aber signifikante Selbsthybridisierung des Konstrukts durch die Verwendung modifizierter Basen und/oder eines modifizierten Gerüstes verhindert wird.

8. Konstrukt nach einem der vorstehenden Ansprüche, wobei intern oder an einem Ende eines der Oligonukleotide zusätzliche Konjugate hinzugefügt werden.

9. Konstrukt nach Anspruch 8, wobei die zusätzlichen Konjugate aus Oligonukleotiden, Peptiden, Proteinen, Markierungen wie Fluoreszenz- oder Isotopmarkierungen, biologisch aktiven oder inerten Molekülen ausgewählt sind.

10. Verwendung eines Konstruktes nach einem der Ansprüche 1 bis 9 für die In-vitro-Translation und - Transkription und Translationshemmung von DNA/RNA durch Stranginvasion doppelsträngiger DNA.

11. Verwendung nach Anspruch 10, wobei die DNA oder RNA eukaryontischen (genomischen), bakteriellen oder viralen Ursprungs ist.

12. Verwendung nach Anspruch 10, wobei die DNA oder RNA ein Tumorgen ist.

13. Verwendung nach Anspruch 10, wobei die DNA oder RNA ein Gen ist, das die Differenzierung von Zellen in Gewebekulturen reguliert.

14. Verwendung eines Konstruktes nach einem der Ansprüche 1 bis 9 für die Herstellung eines Medikaments zum therapeutischen Stummschalten von Genexpression.

15. Verwendung nach Anspruch 14, wobei das Gen ein Tumorgen ist.

16. In-vitro-Verfahren für die Hemmung von Translation und Transkription von DNA oder RNA durch Stranginvasion doppelsträngiger DNA, wobei ein Konstrukt nach einem der Ansprüche 1 bis 9 verwendet wird.

17. Verfahren nach Anspruch 16, wobei die DNA oder RNA eukaryontischen (genomischen), bakteriellen oder viralen Ursprungs ist.

## Revendications

1. Construction comprenant au moins un premier et un second oligonucléotides, reliés l'un à l'autre, où les oligonucléotides sont choisies parmi de l'ADN, de l'ARN, leurs analogues ou l'un quelconque de leurs mélanges, et où la composition des oligonucléotides peut être choisie indépendamment pour les différents oligonucléotides, **caractérisée en ce que** lesdits premier et second oligonucléotides sont constitués de LNA et/ou de PNA et ensemble ont une structure telle qu'illustrée sur la figure 10, lesdits oligonucléotides sont reliés l'un à l'autre par l'intermédiaire de l'appariement des bases de Watson-Crick spécifiques de la séquence, et où ladite construction s'hybride par l'intermédiaire de l'appariement des bases de Watson-Crick spécifiques de la séquence à un ADN double brin d'une façon trans envahissant le brin pour affecter la transcription.

2. Construction selon la revendication 1, dans laquelle dans les cas où plus de deux oligonucléotides sont utilisés, au moins un est relié aux deux extrémités.

3. Construction selon la revendication 1, dans laquelle les séquences cibles oligonucléotidiques sur l'ADN double brin sont en étroite proximité, espacées de moins de 5 bases.

4. Construction selon la revendication 1, dans laquelle les séquences cibles oligonucléotidiques sont adjacentes.

5. Construction selon la revendication 1, dans laquelle les séquences cibles oligonucléotidiques se chevauchent partiellement.

6. Construction selon la revendication 5, dans laquelle les séquences cibles oligonucléotidiques se chevauchent de 1 à 8 bases.

7. Construction selon la revendication 5, dans laquelle les séquences cibles oligonucléotidiques se chevauchent substantiellement ou totalement et une auto-hybridation complète ou partielle, mais significative, de la construction est prohibée par l'utilisation de bases modifiées et/ou d'un squelette modifié.

8. Construction selon l'une quelconque des revendications ci-dessus, dans laquelle des conjugués supplémentaires sont ajoutés en interne ou à l'une ou l'autre extrémité de l'un quelconque des oligonucléotides.

9. Construction selon la revendication 8, dans laquelle lesdits conjugués supplémentaires sont choisis parmi des oligonucléotides, des peptides, des protéines, des marqueurs comme des marqueurs fluorescents ou isotopiques, des molécules biologiquement actives ou inertes.

10. Utilisation d'une construction selon l'une quelconque des revendications 1 à 9, pour la traduction *in vitro* et la transcription et l'inhibition de la traduction d'ADN/ARN par invasion de brin d'ADN double brin.

11. Utilisation selon la revendication 10, où l'ADN ou l'ARN est d'origine eucaryote (génomique), bactérienne ou virale.

12. Utilisation selon la revendication 10, où l'ADN ou l'ARN est un gène tumoral.

13. Utilisation selon la revendication 10, où l'ADN ou l'ARN est un gène régulant la différenciation des cellules dans des cultures tissulaires.

14. Utilisation d'une construction selon l'une quelconque des revendications 1 à 9, pour la fabrication d'un médicament pour le silençage thérapeutique de l'expression de gènes.

15. Utilisation selon la revendication 14, où le gène est un gène tumoral.

16. Procédé *in vitro* pour l'inhibition de la traduction et de la transcription d'ADN ou d'ARN par invasion de brin d'ADN double brin, dans lequel une construction selon l'une quelconque des revendications 1 à 9 est utilisée.

17. Procédé selon la revendication 16, dans lequel l'ADN ou l'ARN est d'origine eucaryote (génomique), bactérienne ou virale.
